# EUROPÄISCHE PATENTSCHRIFT

(11) **EP 2 603 285 B1**
(45) Veröffentlichungstag und Bekanntmachung des Hinweises auf die Patenterteilung: **15.11.2017**
(21) Anmeldenummer: 11743970.3
(22) Anmeldetag: 28.07.2011
(51) Int. Cl.: A61N 1/39, A61N 1/05

(54) **KARDIOVERTER ZUR BESEITIGUNG VON VORHOFFLIMMERN**
CARDIOVERTERS FOR ELIMINATING ATRIAL FIBRILLATION
CARDIOVERTEUR POUR ÉLIMINER LA FIBRILLATION AURICULAIRE

(30) Priorität: 10.08.2010 DE 202010011244 U
(43) Veröffentlichungstag der Anmeldung: 19.06.2013
(73) Patentinhaber: Osypka, Peter, 79639 Grenzach-Wyhlen (DE)
(72) Erfinder: Osypka, Peter, 79639 Grenzach-Wyhlen (DE)
(74) Vertreter: Börjes-Pestalozza, Henrich
(86) Internationale Anmeldenummer: PCT/EP2011/003789
(87) Internationale Veröffentlichungsnummer: WO 2012/019715

(56) Entgegenhaltungen:
- US-A- 5 674 251
- US-A1- 2002 072 775
- US-A1- 2006 217 769

## Beschreibung

Die Erfindung, welche in Anspruch 1 definiert ist, betrifft einen Kardioverter mit einem integrierten Stimulator zur Therapie des Herzens.

Die Erfindung betrifft dabei zwei unabhängig arbeitende elektrische Systeme zur Therapie des Herzens, bestehend aus Kardioverter und integriertem Stimulator. Kardioverter und Stimulator haben dabei jeweils zwei separate Endstufen, die voneinander zeitlich versetzt jeweils einen Energieimpuls abgeben können, aber auch synchron vom Ventrikel EKG synchronisiert werden können.

### Problem:

Postoperatives Vorhofflimmern tritt relativ häufig auf und ist einer der wichtigsten Gründe für die postoperative Morbidität. Insgesamt scheint Vorhofflimmern nach Herzoperationen in den letzten Jahren zugenommen zu haben, was mit einem immer älter werdenden Patientenkollektiv erklärt wird. Literaturangaben über postoperatives Vorhofflimmern bei Patienten mit vorbestehendem Sinusrhythmus ergaben ein durchschnittliches Auftreten in 30 % - 40 % nach Bypassoperationen am Herzen. Vorhofflimmern führt zu einer schnellen Überleitung der Erregung auf die Ventrikel, so dass es zu akuter hämodynamischer Instabilität kommen kann. Die elektrische Kardioversion ist eine nicht-medikamentöse und sehr effektive Methode zur Wiederherstellung des Sinusrhythmus, die allerdings eine Kurznarkose zur Voraussetzung hat. Diese Kurznarkose kann jedoch gerade bei Patienten nach Bypassoperationen die bestehenden neuronalen Probleme (Vigilanz) durch die soeben überstandene Herzoperation verschärfen, was zu einer verlängerten Aufwachphase führen kann oder sogar eine erneute Intubation mit maschineller Beatmung erforderlich machen. Ein weiteres Problem stellt die Antikoagulation bei postoperativen Patienten mit Vorhofflimmern dar. Wenn die Rhythmusstörung länger als 24 Stunden anhält, ist eine Antikoagulation erforderlich, um die Thrombenbildung mit der Gefahr eines Schlaganfalls zu reduzieren. All diese Faktoren führen zu einem komplizieren postoperativen Verlauf bei Patienten nach Bypassoperationen, der sich in einem um ca. 5 Tage verlängerten Krankenhausaufenthalt mit vermehrten Kosten niederschlägt.

### Wie wird das Problem bisher gelöst:

Die Beseitigung von Vorhofflimmern nach einer Herz-Operation erfolgt bisher durch einen äußeren elektrischen Energieimpulse mittels eines Defibrillators, appliziert durch Auflage oder Aufkleben von großflächigen Elektroden auf den Brustkorb des Patienten. Dazu ist es notwendig, dass beim Patienten vorher eine Ultraschalluntersuchung des linken Herzohres durchgeführt werden muss und während der Kardioversion eine Narkose notwendig ist.

Aus der US 2002/0072775 A1 und aus der US 2006/0217769 A1 ist jeweils ein implantierbares Gerät zum Stimulieren und Defibrillieren bekannt, welches aber nicht für eine unmittelbare postoperative Anwendung geeignet ist, weil es für seine Benutzung implantiert werden muss.

### Lösungsweg:

Es besteht daher die Aufgabe, einen Kardioverter zu schaffen, der eine unmittelbare, zuverlässige und möglichst schmerzfreie Beseitigung des Vorhofflimmerns des Herzens ermöglicht.

Diese Aufgabe wird mit den Mitteln und Merkmalen des Patentanspruches 1 gelöst. Vorteilhafte Ausgestaltungen oder Weiterbildungen sind in den Ansprüchen 2 bis 10 enthalten.

Dadurch, dass die Kardioversion gleichzeitig an den beiden Vorhöfen intrakardial und lokal, aber mit separaten Energieimpulsen vorgenommen wird, reduziert sich die notwendige elektrische Energieabgabe zur Kardioversion erheblich. Auf diese Weise ergibt sich die Möglichkeit einer unmittelbaren Beseitigung des Vorhofflimmerns ohne Narkose für den Patienten. Selbstverständlich kann der Kardioverter auch ohne Stimulator betrieben werden.

Eine besonders vorteilhafte Ausführungsform der Erfindung von eigener schutzwürdiger Bedeutung kann darin bestehen, dass der Kardioverter mit dem Stimulator in zwei Teile aufgeteilt ist, dass die beiden Teile jeweils getrennt auf den beiden Vorhöfen des Herzens fixierbare, insbesondere bipolare Elektroden aufweisen und dass die Kardioversion in Gebrauchslage gleichzeitig an den beiden Vorhöfen intrakardial und lokal aber mit separaten Energieimpulsen durchführbar ist. Der Kardioverter und sein Stimulator haben jeweils zwei separate Endstufen, die mit getrennten bipolaren Elektroden zum Fixieren an den Außenseiten der Herzvorhöfe verbunden sind. Dadurch wird die schon erwähnte unmittelbare Beseitigung des Vorhofflimmerns ohne Narkose auf einfache Weise ermöglicht.

Der Kardioverter weist durch die Aufteilung in zwei Teile zwei Ausgänge und von diesen ausgehende bipolare epikardiale Elektroden auf, ist also einfach in seinem Aufbau.

Dabei sind zweckmäßigerweise Mittel dafür vorgesehen, dass die abgegebenen Schockimpulse für eine Defibrillation oder Kardioversion der beiden Vorhöfe des Herzen zeitlich versetzt sind. Zwar können diese Schockimpulse gleichzeitig, aber eben auch zeitlich versetzt zur Anwendung kommen.

Zweckmäßige Größen der jeweiligen Energieimpulse für die Kardioversion sind in den Ansprüchen 7 und 8 angegeben.

Die angestrebte Aufgabe wird besonders gut durch die Ausgestaltung gemäß Anspruch 10 gelöst, wonach nämlich die beiden Elektroden in den Vorhöfen und die Ausgänge der Kardioverter so schaltbar oder geschaltet sind, dass die Energieimpulse für die Kardioversion mit entsprechender Polarität zwischen den beiden Vorhöfen des Herzens wirksam sind.

Eine weitere Möglichkeit kann darin bestehen, dass die Ausgänge der Kardioverter so schaltbar oder geschaltet sind, dass die Energieimpulse für die Kardioversion für die beiden Vorhöfe des Herzens telemetrisch übertragen werden oder übertragbar sind.

Eine weitere Möglichkeit oder Ausgestaltung kann vorsehen, dass die Ausgänge der Kardioverter beziehungsweise der beiden Teile des Kardioverters so geschaltet oder schaltbar sind, dass die Energieimpulse für die Kardioversion zwischen Vorhof und einer äußeren indiffernten Elektrode abgegeben werden können.

Ferner ist es möglich, dass die Energieimpulse für die Kardioversion zwischen Vorhof und einer indifferenten Elektrode im Oesophagus abgegeben werden können.

Eine zweckmäßige Ausgestaltung der Erfindung kann vorsehen, dass eine weitere bipolare Sensing-Elektrode vorgesehen ist. Somit kann die Herztätigkeit durch eine solche Sensing-Elektrode überwacht werden.

Das ganze System kann durch das Ventrikel-EKG getriggert beziehungsweise synchronisiert sein, insbesondere mittels der bipolaren Sensing-Elektrode.

Weitere Einzelheiten, Merkmale und Vorteile der Erfindung lassen sich dem folgenden Beschreibungsteil entnehmen, in dem anhand der Zeichnung und des Ausführungsbeispiels die Erfindung näher erläutert wird.

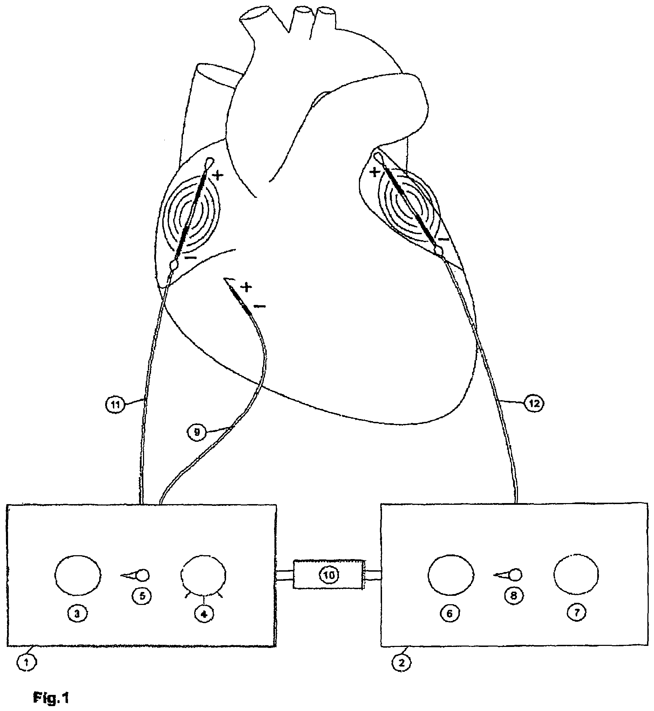

Fig. 1 zeigt in schematischer Darstellung einen Kardioverter / Stimulator (aufgeteilt in (1) und (2)), jeweils getrennt für die beiden Vorhöfe mit den entsprechenden Elektroden (11) und (12) am Herzen.

### Beschreibung der Abbildung:

Fig. 1 zeigt zwei bipolare Elektroden 11 und 12, die auf den beiden Vorhöfen des Herzens fixiert sind und die mit einem Kardioverter, der gleichzeitig auch einen Stimulator enthält (aufgeteilt in (1) und (2)), verbunden sind. Der Kardioverter kann zwei biphasische Energieimpulse synchron und gleichzeitig oder mit einer durch eine Einstellvorrichtung 10 einstellbaren Verzögerung von beispielsweise O - 500 ms, abgeben. Die beiden Ausgänge des Kardioverters sind getrennt isoliert aufgebaut, so dass sich die Ausgangssignale in keiner Weise beeinflussen können. Die von jedem Ausgang abzugebende Energie kann in einem Bereich zwischen beispielsweise 1 bis 20 Joule durch die Einstellvorrichtungen 3 und 6 eingestellt werden; aber auch größere Energieabgaben sind möglich. Stimulationsamplitude, Stimulationsfrequenz und Stimulationsempfindlichkeit können durch Einstellvorrichtungen 4 und 7 eingestellt werden, wobei diese getrennt oder als eine Einheit aufgeführt sein können.

Nach jeder Kardiovertierung aber auch während des vorhofflimmerfreien Zustands des Herzens können die Vorhöfe des Herzens elektrisch stimuliert werden. Dazu wird der Kardioverter durch die Umschaltvorrichtungen 5 und 8 umgeschaltet und der ebenfalls darin enthaltene Stimulator aktiviert. Der Stimulator ist je nach Bedarf in der Lage, die beiden Vorhöfe und Ventrikel des Herzens in der üblichen Weise zu stimulieren. Das ganze System kann durch das Ventrikel EKG (QRS-Komplex) getriggert beziehungsweise synchronisiert werden. Hierfür ist in Fig. 1 beispielhaft eine weitere bipolare Sensing-Elektrode 9 dargestellt.

Die beiden Teile des Kardioverters und somit die beiden bipolaren Elektroden auf den Vorhöfen können auch so geschaltet werden, dass die Energieabgabe zwischen den beiden Vorhöfen erfolgt. Darüber hinaus kann durch den getrennt isolierten Aufbau beispielsweise der rechte Vorhof stimuliert werden, während der linke Vorhof kardiovertiert wird. Dabei wird man besonders auf die Stimulation der Vorhöfe des Herzens achten, um eine eventuelle Wiederkehr des Vorhofflimmerns zu vermeiden. Sollte eine Therapie des Vorhofflimmerns auf diese Weise nicht zu einem nachhaltigen Erfolg führen und der Patient sich z.B. einer RF Ablation unterziehen müssen, so kann die Energieapplikation auch telemetrisch über geeignete Spulen über einen längeren Zeitraum weiterhin fortgesetzt werden (hier nicht dargestellt). Die telemetrische Übertragung von Energie und Informationen ist Stand der Technik und soll hier nicht näher erläutert werden. Die Position, die Art der Ausführung und die Fixierung der Spulen kann dabei je nach der Anatomie an verschiedenen Teilen im menschlichen Körper implantiert werden, so dass eine spätere Explanation leicht durchgeführt werden kann. Auch der Kardioverter und der Stimulator müssen mit entsprechenden Sendespulen ausgerüstet sein.

Beispielsweise kann der Kardioverter mit Batterie für diesen Zeitraum extern am Gürtel des Patienten positioniert sein.

Eine weitere Art der Kardiovertierung kann dadurch erfolgen, dass die Energieimpulse gegen eine gemeinsame indifferente Außenelektrode (hier nicht dargestellt) appliziert werden.

Eine weitere Art der Kardiovertierung kann dadurch erfolgen, dass die Energieimpulse gegen eine gemeinsame indifferente Elektrode, die sich als Katheter mit entsprechender großflächiger Elektrode im Oesophagus befindet (hier nicht dargestellt), appliziert werden.

## Patentansprüche

1. Kardioverter mit integriertem Stimulator zur Therapie des Herzens, wobei der Kardioverter mit dem Stimulator in zwei Teile (1, 2) aufgeteilt ist, und der Kardioverter durch die Aufteilung in zwei Teile (1, 2) zwei Ausgänge besitzt, wobei jeder Teil jeweils einen der besagten Ausgänge besitzt, wobei zwei bipolare Elektrodenleitungen (11) und (12), dessen jeweils zwei Elektroden auf den beiden Vorhöfen fixierbar sind, jeweils mit besagten Ausgängen des Kardioverters verbunden sind, und wobei die beiden Ausgänge getrennt isoliert aufgebaut sind, so dass sich die Ausgangssignale in keiner Weise beeinflussen können, und wobei der Kardioverter zwei Einstellvorrichtungen (3) und (6) aufweist zum Einstellen der von jedem Ausgang abzugebenden Energie, und wobei der Kardioverter zwei Einstellvorrichtungen (4) und (7) aufweist zum Einstellen der Stimulationsamplitude, Stimulationsfrequenz und Stimulationsempfindlichkeit, und wobei der Konverter zwei Umschaltvorrichtungen (5) und (8) aufweist zur Aktivierung des im Kardioverter integrierten Stimulators und wobei besagte Teile des Kardioverters zwei biphasische Engergieimpulse synchron und gleichzeitig oder mit einer durch eine Einstellvorrichtung (10) einstellbaren Verzögerung abgeben kann.

2. Kardioverter mit Stimulator nach Anspruch 1, **dadurch gekennzeichnet, dass** die jeweiligen Energieimpulse für die Kardioversion zwischen 0 und 20 Joule einstellbar sind.

3. Kardioverter mit Stimulator nach einem der Ansprüche 1 oder 2, **dadurch gekennzeichnet, dass** die jeweiligen Energieimpulse für die Stimulation zwischen 0 und 30 Volt und einer Impulsbreie zwischen 0,5 ms und 20 ms einstellbar sind.

4. Kardioverter mit Stimulator nach einem der Ansprüche 1 bis 3, **dadurch gekennzeichnet, dass** die Energieabgaben für den linken Vorhof zwischen 0 und 500 ms verzögert gegenüber dem rechten Vorhof einstellbar sind.

5. Kardioverter und Stimulator nach einem der Ansprüche 1 bis 4, **dadurch gekennzeichnet, dass** die beiden bipolaren Elektroden (11, 12) an den Vorhöfen und die Ausgänge der Kardioverter (1, 2) so schaltbar oder geschaltet sind, dass die Energieimpulse für die Kardioversion mit entsprechender Polarität an den beiden Vorhöfen gleichzeitig aber mit separaten Energieimpulsen durchführbar sind.

6. Kardioverter nach einem der vorstehenden Ansprüche, **dadurch gekennzeichnet, dass** die beiden epikardialen Elektroden (11 und 12) an den Vorhöfen und die Ausgänge der Kardioverter so schaltbar oder geschaltet sind, dass die Energieimpulse für die Kardioversion mit entsprechender Polarität zwischen den beiden Vorhöfen des Herzens wirksam sind.

7. Kardioverter und Stimulator nach einem der Ansprüche 1 bis 6, **dadurch gekennzeichnet, dass** die Ausgänge der Kardioverter (1, 2) so schaltbar oder geschaltet sind, dass die Energieimpulse für die Kardioversion für die beiden Vorhöfe des Herzens telemetrisch übertragen werden.

8. Kardioverter und Stimulator nach einem der Ansprüche 1 bis 7, **dadurch gekennzeichnet, dass** die Ausgänge der Kardioverter (1, 2) so schaltbar oder geschaltet sind, dass die Energieimpulse für die Kardioversion zwischen Vorhof und einer äußeren indifferenten Elektrode abgegeben werden können.

9. Kardioverter und Stimulator nach einem der Ansprüche 1 bis 8, **dadurch gekennzeichnet, dass** die Energieimpulse für die Kardioversion zwischen Vorhof und einer indifferenten Elektrode im Oesophagus abgegeben werden können.

10. Kardioverter und Stimulator nach einem der Ansprüche 1 bis 9, **dadurch gekennzeichnet, dass** eine weitere bipolare Sensing-Elektrode (9) vorgesehen ist, die vorzugsweise den Ablauf der Kardioversion steuert beziehungsweise synchronisiert.

## Claims

1. Cardioverter comprising an integrated stimulator for therapy of the heart, wherein the cardioverter with the stimulator is divided into two parts (1, 2), and that the cardioverter, by being divided into two parts (1, 2), has two outputs, wherein each part respectively has one of said outputs, wherein two bipolar electrode lines (11) and (12), of which in each case two electrodes can be attached to the two atria, are each connected to said outputs of the cardioverter, and wherein the two outputs are constructed separately in an isolated manner, so that the output signals cannot influence one another in any way, and wherein the cardioverter has two setting devices (3) and (6) for setting the energy to be emitted by each output, and wherein the cardioverter has two setting devices (4) and (7) for setting the stimulation amplitude, stimulation frequency and stimulation sensitivity, and wherein the converter has two switching devices (5) and (8) for activating the stimulator integrated in the cardioverter, and wherein said parts of the cardioverter can emit two biphasic energy pulses synchronously and simultaneously or with a delay which can be set by a setting device (10).

2. Cardioverter comprising a stimulator as claimed in claim 1, **characterised in that** the respective energy pulses for the cardioversion can be set between 0 and 20 joules.

3. Cardioverter comprising a stimulator as claimed in any one of claims 1 or 2, **characterised in that** the respective energy pulses for the stimulation can be set between 0 and 30 volts and a pulse width of between 0.5 ms and 20 ms.

4. Cardioverter comprising a stimulator as claimed in one of claims 1 to 3, **characterised in that** the energy emissions for the left atrium can be set to have a delay of between 0 and 500 ms compared to the right atrium.

5. Cardioverter and stimulator as claimed in any one of claims 1 to 4, **characterised in that** the two bipolar electrodes (11, 12) at the atria and outputs of the cardioverters (1, 2) can be connected or are connected in such a way that the energy pulses for the cardioversion can be performed, with corresponding polarity, simultaneously but with separate energy pulses at the two atria.

6. Cardioverter as claimed in one of the preceding claims, **characterised in that** the two epicardial electrodes (11 and 12) at the atria and the outputs of the cardioverters can be connected or are connected in such a way that the energy pulses for the cardioversion are effective, with corresponding polarity, between the two atria of the heart.

7. Cardioverter and stimulator as claimed in any one of claims 1 to 6, **characterised in that** the outputs of the cardioverters (1, 2) can be connected or are connected in such a way that the energy pulses for the cardioversion for the two atria of the heart are transmitted telemetrically.

8. Cardioverter and stimulator as claimed in any one of claims 1 to 7, **characterised in that** the outputs of the cardioverters (1, 2) can be connected or are connected in such a way that the energy pulses for the cardioversion can be emitted between the atrium and an external neutral electrode.

9. Cardioverter and stimulator as claimed in any one of claims 1 to 8, **characterised in that** the energy pulses for the cardioversion can be emitted between the atrium and a neutral electrode in the oesophagus.

10. Cardioverter and stimulator as claimed in any one of claims 1 to 9, **characterised in in that** a further bipolar sensing electrode (9) is provided which preferably controls or synchronizes the sequence of the cardioversion.

## Revendications

1. Cardioverteur avec stimulateur intégré pour la thérapie du coeur, dans lequel le cardioverteur avec le stimulateur est divisé en deux parties (1, 2), dans lequel le cardioverteur comporte deux sorties du fait de la division en deux parties (1, 2), dans lequel chaque partie comporte respectivement une desdites sorties, dans lequel deux conducteurs d'électrode bipolaires (11) et (12), dont deux électrodes peuvent être respectivement fixées sur les deux oreillettes, sont respectivement connectés auxdites sorties du cardioverteur, et dans lequel les deux sorties sont construites séparément de façon isolée, de telle manière que les signaux des sorties ne puissent en aucune façon s'influencer, et dans lequel le cardioverteur présente deux dispositifs de réglage (3) et (6) pour le réglage de l'énergie à produire par chaque sortie, et dans lequel le cardioverteur présente deux dispositifs de réglage (4) et (7) pour le réglage de l'amplitude de stimulation, de la fréquence de stimulation et de la sensibilité de stimulation, et dans lequel le convertisseur présente deux dispositifs d'inversion (5) et (8) pour l'activation du stimulateur intégré dans le cardioverteur et dans lequel lesdites parties du cardioverteur peuvent produire des impulsions d'énergie biphasées de façon synchrone et simultanée ou avec un retard réglable par un dispositif de réglage (10).

2. Cardioverteur avec stimulateur selon la revendication 1, **caractérisé en ce que** les impulsions d'énergie respectives pour la cardioversion sont réglables entre 0 et 20 joules.

3. Cardioverteur avec stimulateur selon une des revendications 1 ou 2, **caractérisé en ce que** les impulsions d'énergie respectives pour la stimulation sont réglables entre 0 et 30 volts et une largeur d'impulsion entre 0,5 ms et 20 ms.

4. Cardioverteur avec stimulateur selon l'une quelconque des revendications 1 à 3, **caractérisé en ce que** les émissions d'énergie pour l'oreillette gauche sont réglables avec un retard entre 0 et 500 ms par rapport à l'oreillette droite.

5. Cardioverteur et stimulateur selon l'une quelconque des revendications 1 à 4, **caractérisé en ce que** les deux électrodes bipolaires (11, 12) sur les oreillettes et les sorties des cardioverteurs (1, 2) peuvent être ou sont connectées de telle manière que les impulsions d'énergie pour la cardioversion peuvent être réalisées avec une polarité correspondante aux deux oreillettes de façon simultanée mais avec des impulsions d'énergie séparées.

6. Cardioverteur selon l'une quelconque des revendications précédentes, **caractérisé en ce que** les deux électrodes épicardiques (11 et 12) sur les oreillettes et les sorties des cardioverteurs peuvent être ou sont connectées de telle manière que les impulsions d'énergie pour la cardioversion soient actives avec une polarité correspondante entre les deux oreillettes du coeur.

7. Cardioverteur et stimulateur selon l'une quelconque des revendications 1 à 6, **caractérisé en ce que** les sorties des cardioverteurs (1, 2) peuvent être ou sont connectées de telle manière que les impulsions d'énergie pour la cardioversion soient transmises par télémétrie pour les deux oreillettes du coeur.

8. Cardioverteur et stimulateur selon l'une quelconque des revendications 1 à 7, **caractérisé en ce que** les sorties des cardioverteurs (1, 2) peuvent être ou sont connectées de telle manière que les impulsions d'énergie pour la cardioversion puissent être émises entre une oreillette et une électrode extérieure indifférente.

9. Cardioverteur et stimulateur selon l'une quelconque des revendications 1 à 8, **caractérisé en ce que** les impulsions d'énergie pour la cardioversion peuvent être émises entre une oreillette et une électrode indifférente dans l'oesophage.

10. Cardioverteur et stimulateur selon l'une quelconque des revendications 1 à 9, **caractérisé en ce qu'**il est prévu une autre électrode de détection bipolaire (9), qui de préférence commande ou synchronise le déroulement de la cardioversion.
